# EUROPÄISCHE PATENTANMELDUNG

(11) **EP 3 964 825 A1**
(43) Veröffentlichungstag der Anmeldung: **09.03.2022**
(21) Anmeldenummer: 21189320.1
(22) Anmeldetag: 03.08.2021
(51) Int. Cl.: G01N 25/18, G01N 33/00, G01N 27/18

(54) **VERFAHREN ZUR ERFASSUNG MINDESTENS EINER EIGENSCHAFT EINES FLUIDEN MEDIUMS IN MINDESTENS EINEM MESSRAUM**

(30) Priorität: 03.09.2020 DE 102020211102
(71) Anmelder: Robert Bosch GmbH, 70442 Stuttgart (DE)
(72) Erfinder: Muellers, Johannes, 70176 Stuttgart (DE)

(57) **Zusammenfassung**

Es wird ein Verfahren zum Kalibrieren eines Sensors (10) zur Erfassung mindestens einer Eigenschaft eines fluiden Mediums (12) in mindestens einem Messraum (14), insbesondere zur Erfassung eines H₂-Anteils in einem Messgas (16), vorgeschlagen, wobei der Sensor (10) ein Sensorelement (18) mit mindestens einer beheizbaren Membran (20) aufweist, wobei die Membran (20) mit einer elektrischen Messbrücke (48) verbunden ist. Das Verfahren umfasst die folgenden Schritte: Anlegen einer elektrischen Heizspannung (U_{H}) an die Membran (20) zum Beheizen der Membran (20), Variieren der Heizspannung (U_{H}) über einen vorbestimmten Zeitraum, Messen einer elektrischen Brückenspannung (U_{B}) an der Messbrücke (48) über den vorbestimmten Zeitraum, Auswerten des zeitlichen Verlaufs der gemessenen Brückenspannung (U_{B}) mittels eines Algorithmus (60), und Ermitteln eines funktionalen Zusammenhangs zwischen der Eigenschaft eines fluiden Mediums (12) und des ausgewerteten zeitlichen Verlaufs der gemessenen Brückenspannung (U_{B}).

## Beschreibung

### Stand der Technik

Aus dem Stand der Technik ist eine Vielzahl von Sensoren, Sensorelementen und Verfahren zur Erfassung mindestens einer Eigenschaft eines fluiden Mediums in einem Messraum bekannt. Dabei kann es sich grundsätzlich um beliebige Eigenschaften eines gasförmigen oder flüssigen fluiden Mediums handeln, wobei eine oder mehrere Eigenschaften erfasst werden können. Die Erfindung wird im Folgenden, ohne Beschränkung weiterer Ausführungsformen und Anwendungen, insbesondere unter Bezugnahme auf Sensoren zur Erfassung eines Gases, insbesondere eines H₂-Anteils in einem Messgas, beschrieben.

Sensoren der hier beschriebenen Art finden Anwendung in einer Vielzahl von Gebieten, beispielsweise in der Automobiltechnik, der Verfahrenstechnik, der Chemie und dem Maschinenbau, insbesondere zur Bestimmung von Gaskonzentrationen. So spielt beispielsweise die Bestimmung von Wasserstoffkonzentrationen, beispielsweise in einem Luft-Wasserstoff-Gemisch, bei der Anwendung von Wasserstoff-Brennstoffzellen-Systemen eine große Rolle. Hierbei sind auch sicherheitsrelevante Anwendungen zu nennen. Ein Luft-Wasserstoff-Gemisch wird etwa bei einem Wasserstoffanteil von 4 % zündungsfähig. Sensoren zur Erfassung von Wasserstoff können beispielsweise in Wasserstoff-Brennstoffzellenfahrzeugen zum Einsatz kommen, um beispielsweise aufgrund von Beschädigung oder Defekt austretenden Wasserstoff zu detektieren und, durch eine Kopplung an entsprechende Systeme, Warnsignale und/oder Schutzmaßnahmen auszulösen. Daher werden pro Brennstoffzellenfahrzeug mehrere Wasserstoffsensoren benötigt, die entweder im Abgasstrang angebracht werden (exhaust) oder unter atmosphärischen Bedingungen arbeiten (ambient).

Für derartige Wasserstoffsensoren kann man auf eine Vielzahl von Messprinzipien zurückgreifen. Dazu gehören u.a. folgende Messprinzipien: Wärmeleitung, katalytischer Pellistor, elektrochemische Zelle, halbleitendes Metalloxid, Chemiresistor, Feldeffekt Transistor.

Aus der DE 10 2005 058 830A1 und DE 10 2005 058 832 A1 ist jeweils ein Sensor zur Erfassung einer Komponente eines mehrere Komponenten enthaltenden gasförmigen Fluids bekannt. Der Sensor umfasst ein Gehäuse, welches einen Messraum begrenzt und in dem ein Messchip mit einer beheizbaren Membran aufgenommen ist. Das Prinzip des beheizten Wasserstoff-Sensors beruht auf der unterschiedlichen Wärmeleitfähigkeit der beteiligten Gaskomponenten. Eine dadurch erzeugte Asymmetrie im thermischen System wird in ein Spannungssignal umgesetzt.

Trotz der Vorteile der aus dem Stand der Technik bekannten Sensoren zur Erfassung mindestens einer Eigenschaft eines fluiden Mediums beinhalten diese noch Verbesserungspotenzial. Um eine hinreichende Messgenauigkeit für Wasserstoff zu erzielen, müssen die Einflüsse von Wasserstoff und anderen Gasbestandteilen, wie beispielsweise Feuchte und Sauerstoff, separiert werden, was durch die Messung bei verschiedenen Heizspannungen geschieht. In das thermische und elektrische System gehen dabei diverse Systemgrößen, wie beispielsweise Membrandicke, elektrische Widerstände usw., ein. Zur Kalibrierung müssen zahlreiche Messungen mit verschiedenen Gas-Zusammensetzungen bei verschiedenen Temperaturen durchgeführt werden. Im Ergebnis entsteht ein mathematisches Modell, das zu gemessenen Spannungen U_{B} die Wasserstoffkonzentration liefert. Die zahlreichen Modell-Parameter werden durch die aufwendige Kalibrierung (Modell-Training) ermittelt. Aufgrund der Bauteil-Toleranzen ändern sich diese Parameter aber von Teil zu Teil. Von zentraler Bedeutung für die Separation der Gas-Quereinflüsse ist die mögliche Genauigkeit der Spannungsmessung. Zur Erhöhung der Abbildungsgenauigkeit bietet sich zunächst die Möglichkeit, bei weiteren Heizspannungen zu messen und so ein überbestimmtes System zu bilden, das mehr Spannungen als Gas-Komponenten beinhaltet. Zusammenfassend sind die bisherigen Kalibrierungsansätze für derartige Sensoren bereits aufwändig oder können zur Verbesserung der Genauigkeit nur sehr aufwändig verändert werden.

### Offenbarung der Erfindung

Im Rahmen der vorliegenden Erfindung wird daher ein Verfahren zum Kalibrieren eines Sensors zur Erfassung mindestens einer Eigenschaft eines fluiden Mediums in einem Messraum vorgeschlagen, welches die Nachteile bekannter Verfahren zum Kalibrieren von Sensoren zur Erfassung mindestens einer Eigenschaft eines fluiden Mediums in einem Messraum zumindest weitgehend vermeidet, und das eine sehr hohe Messgenauigkeit bei weniger Messaufwand liefert.

Unter dem Ausdruck "Kalibrieren" kann hierbei insbesondere eine Messung verstanden werden, welche insbesondere zu einer Feststellung und/oder zu einer Dokumentation und/oder zu einem Ausgleich einer Abweichung eines Verhaltens des Sensors, insbesondere des Sensorelements, zu einem anderen Sensor verwendet werden kann. Derartige Abweichungen können insbesondere Abweichungen zu einem anderen Sensor bzw. Sensorelement und/oder einer Norm und/oder einem Mittelwert sein. Abweichungen können beispielsweise aufgrund von Fertigungsstreuungen auftreten. Weiterhin kann das "Kalibrieren" eine Berücksichtigung der ermittelten Abweichung, insbesondere während einer Benutzung des Sensors, insbesondere des Sensorelements, umfassen, beispielsweise bei der Erfassung des Anteils der Zielgaskomponente. Die Kalibration kann weiterhin einen Kennlinienabgleich umfassen, wobei unter einer Kennlinie beispielsweise ein Zusammenhang zwischen einer Messgröße, beispielsweise einer gemessenen elektrischen Spannung, und einem Anteil mindestens einer Zielgaskomponente verstanden werden kann. Wasserstoffsensoren kann es sich bei der Kennlinie insbesondere um einen funktionalen Zusammenhang zwischen einer gemessenen Brückenspannung einer Messbrücke des Sensorelements und einer Wasserstoffkonzentration handeln.

Ein erfindungsgemäßes Verfahren zum Kalibrieren eines Sensors zur Erfassung mindestens einer Eigenschaft eines fluiden Mediums in mindestens einem Messraum, insbesondere zur Erfassung eines H₂-Anteils in einem Messgas, wobei der Sensor ein Sensorelement mit mindestens einer beheizbaren Membran aufweist, wobei die Membran mit einer elektrischen Messbrücke verbunden ist, umfasst die folgenden Schritte:
- Anlegen einer elektrischen Heizspannung an die Membran zum Beheizen der Membran,
- Variieren der Heizspannung über einen vorbestimmten Zeitraum,
- Messen einer elektrischen Brückenspannung an der Messbrücke über den vorbestimmten Zeitraum,
- Auswerten des zeitlichen Verlaufs der gemessenen Brückenspannung mittels eines Algorithmus, und
- Ermitteln eines funktionalen Zusammenhangs zwischen der Eigenschaft eines fluiden Mediums und des ausgewerteten zeitlichen Verlaufs der gemessenen Brückenspannung.

Unter Messen kann insbesondere eine Messung verstanden werden, bei welcher die Eigenschaft des fluiden Mediums quantitativ ermittelt wird. Beispielsweise wird der Anteil von mindestens einer Zielgaskomponente wie H2 eines Gases wie Luft in dem Messraum quantitativ ermittelt wird. Bei dem Anteil der mindestens einen Zielgaskomponente kann es sich beispielsweise um einen Partialdruck und/oder einen Prozentsatz der Zielgaskomponente handeln. Bei dem Anteil der Zielgaskomponente kann es sich beispielsweise auch um mehrere Anteile einer Zielgaskomponente handeln. Bei der Zielgaskomponente kann es sich prinzipiell um jedes beliebige Gas handeln, dessen Anteil in dem Gas erfasst werden kann. Bevorzugt kann es sich bei der Zielgaskomponente um Wasserstoff handeln. Bei dem Gas kann es sich prinzipiell um ein beliebiges Gas, insbesondere ein Gasgemisch handeln. Bei dem Gas kann es sich bevorzugt um Luft handeln, insbesondere im Kraftfahrzeugbereich. Bei dem Messraum kann es sich beispielsweise um eine Gaszelle des Sensors handeln. Prinzipiell kann unter einem Messraum ein Raum verstanden werden, in welchem sich das fluide Medium bzw. Gas befindet und so die Membran kontaktiert.

Im Gegensatz zu herkömmlichen Kalibrierungsverfahren kann man während derselben Messzeit auch einen variierenden Heizspannungsverlauf aufgeben und das resultierende instationäre Spannungssignal U_{B}(t) auswerten. Dieses umfasst nun so viele Werte, wie man zeitlich abtastet. Dieses Spannungssignal wird einem Algorithmus zugeführt und ausgewertet. Dieses bildet somit einen gemessenen Spannungsverlauf auf beispielsweise eine Wasserstoff-Konzentration ab: U_{B}(t) → yH₂. Da hierbei über den Zeitverlauf sehr viele Stützstellen vorliegen, umfasst das Modell nicht nur die Separation der Gas-Komponenten, sondern auch die Teile-Teile-Streuung - ja sogar die Temperatur des Systems. Und die erforderliche Spannungsungenauigkeit ist ebenfalls geringer als bei der diskreten Messung. In der Fertigung erübrigt sich dadurch eine weitere Kalibrierung, bzw. es genügen einige wenige Validierungsmessungen. Diese wiederum können dazu genutzt werden, den Algorithmus weiter zu trainieren.

Die Heizspannung kann stetig variiert werden. Insbesondere kann die Heizspannung periodisch variiert werden. Somit wird die Heizspannung nicht sprunghaft oder stufenförmig variiert, sondern allmählich mit ggf. einem periodischen Ablauf. Durch geeignete Wahl des Zeitraums, erhält man rasch z.B. nach einer Periode einen eingeschwungenen Zustand und somit einen quasi stationären Verlauf. Dies erlaubt eine zuverlässige und einfache Signalauswertung.

Der Algorithmus kann ein künstlich neuronales Netz umfassen. Dabei wird erfindungsgemäß von den Methoden des Transfer-Learnings gebraucht gemacht, in der Art, dass das komplette zeitaufwändige Training des neuronalen Netzes vorab (außerhalb der Serienfertigung) an einer hinreichenden Anzahl von Produktsensoren erfolgt. Die Sensor-Produkte erhalten diese Knoten-Parameter bei ihrer Programmierung während der Serien-Fertigung, und durchlaufen nur ein kurzes Training zum Zweck eines individuellen Fine-Tunings. Das Training besteht darin, den Sensor nacheinander Luft mit verschiedenen Anteilen an Gasen wie vor allem H₂ bei ggf. verschiedenen Temperaturen auszusetzen, und die eingestellten Parameter für die Gaskonzentrationen als Trainingsdaten für das neuronale Netz zu verwenden. Das neuronale Netz erhält dabei die Daten zur Brückenspannung in Abhängigkeit von der Zeit, d.h. den zeitlichen Verlauf der gemessenen Brückenspannung.

Die elektrische Brückenspannung kann nach einem Einschwingen eines die Brückenspannung anzeigenden Messsignals gemessen werden. Hier kann somit ein quasi stationäres Signal erfasst und ausgewertet werden, was die Messgenauigkeit erhöht.

Eine Länge des vorbestimmten Zeitraums kann basierend auf einer physikalischen Zeitskala des Messraums bestimmt werden. Ein zu kurzer Zeitraum ergibt einen geringen Signalhub und eine zu große maximale Membrantemperatur. Ein zu langer Zeitraum erhöht dagegen unnötig die gesamte erforderliche Messzeit.

Die physikalische Zeitskala kann basierend auf einer geometrischen Längenskala, Dichte, spezifische Wärmekapazität und Wärmeleitfähigkeit des Messraums bestimmt werden. Damit kann der Zeitraum gut abgeschätzt werden. So kann man die physikalische Zeitskala für das thermische System aus geometrischer Längenskala, Dichte, spezifische Wärmekapazität und Wärmeleitfähigkeit der Gaszelle abschätzen: τ = I² ρ cₚ/λ.

Die Länge des vorbestimmten Zeitraums kann 2 bis 20 mal und bevorzugt 5 bis 15 mal größer als die physikalische Zeitskala sein. Die Länge des Zeitraums für die Messung beträgt beispielsweise sinnvollerweise das ca. 10-fache der physikalischen Zeitskala. Genaueres kann man aus Simulationen oder Messungen ableiten. Eine zu kurze Signalperiode ergibt einen geringen Signalhub und eine zu große maximale Membrantemperatur. Eine zu lange Periode erhöht dagegen unnötig die gesamte erforderliche Messzeit.

Die Schritte des Verfahrens können bei unterschiedlichen Temperaturen in dem Messraum wiederholt werden. Mit anderen Worten kann das Verfahren unterschiedliche Temperaturen berücksichtigen, indem die Schritte des Verfahrens nach Ablauf des Verfahrens bei einer Temperatur bei weiteren, unterschiedlichen Temperaturen durchgeführt werden.

Das Verfahren kann bei mehreren verschiedenen Sensoren durchgeführt werden, wobei der zeitliche Verlauf der gemessenen Brückenspannung der verschiedenen Sensoren mittels des Algorithmus ausgewertet wird, wobei der funktionale Zusammenhang zwischen der Eigenschaft eines fluiden Mediums und des ausgewerteten zeitlichen Verlaufs der gemessenen Brückenspannung für jeden Sensor ermittelt wird.

Somit kann im Labor eine genügende Anzahl von Musterteilen in möglichst breiten Toleranzlagen mittels einer DOE für verschiedene Temperaturen und Gas-Zusammensetzungen vermessen werden. Anschließend wird aus den gesamten Messdaten (Spannungsverlauf U_{B,ik} (t) für das Teil i und den Versuch k und daraus ermittelt Wasserstoff-Konzentration yH_{2,k}) ein neuronales Netz trainiert. Dieses bildet somit einen gemessenen Spannungsverlauf auf eine Wasserstoff-Konzentration ab: U_{B}(t) → yH₂. Da hierbei über den Zeitverlauf sehr viele Stützstellen vorliegen, umfasst das Modell nicht nur die Separation der Gas-Komponenten, sondern auch die Teile-Teile-Streuung - ja sogar die Temperatur des Systems. Und die erforderliche Spannungsgenauigkeit ist ebenfalls geringer als bei der diskreten Messung.

### Kurze Beschreibung der Zeichnungen

Weitere optionale Einzelheiten und Merkmale der Erfindung ergeben sich aus der nachfolgenden Beschreibung bevorzugter Ausführungsbeispiele, welche in den Figuren schematisch dargestellt sind.

Es zeigen:
Figur 1 eine schematische Darstellung eines Sensors zur Erfassung mindestens einer Eigenschaft eines fluiden Mediums in mindestens einem Messraum, und
Figur 2 eine schematische Darstellung eines elektrischen Schaltbilds des Sensorelements des Sensors,
Figur 3 einen beispielhaften zeitlichen Verlauf von Heizspannung und Brückenspannung,
Figur 4 eine schematische Darstellung eines beispielhaften Algorithmus zur Verwendung bei dem erfindungsgemäßen Verfahren,
Figur 5 einen Teil einer Modifikation des erfindungsgemäßen Verfahrens und
Figur 6 einen weiteren Teil der Modifikation des erfindungsgemäßen Verfahrens.

### Ausführungsformen der Erfindung

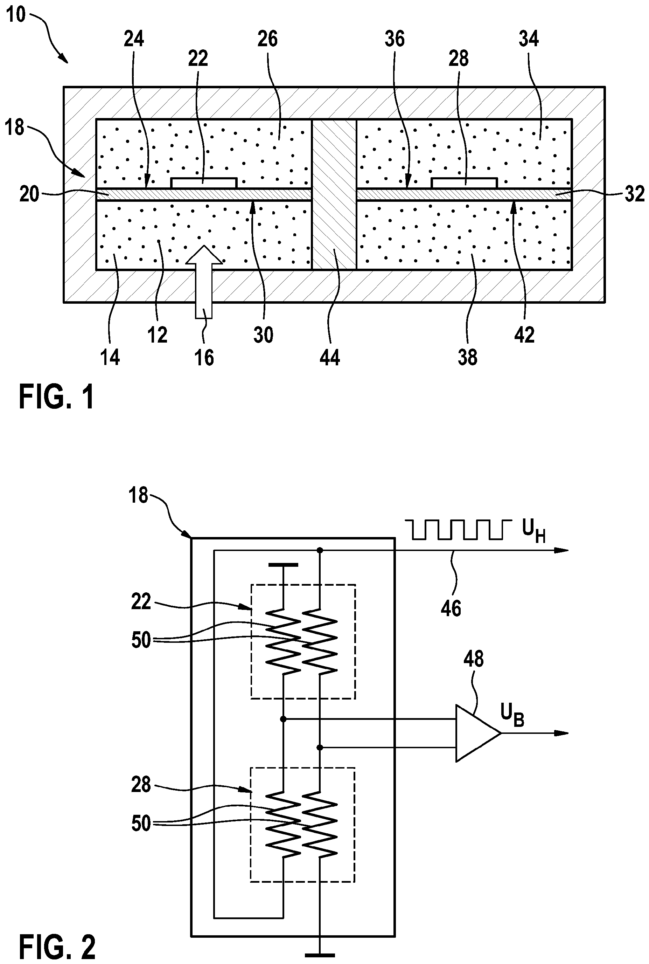

Figur 1 zeigt eine schematische Darstellung eines Sensors 10 zur Erfassung mindestens einer Eigenschaft eines fluiden Mediums 12 in mindestens einem Messraum 14, insbesondere zur Erfassung eines H₂-Anteils in einem Messgas 16. Der Sensor 10 kann insbesondere zum Einsatz in einem Wasserstoff-Brennstoffzellenfahrzeug eingerichtet sein. Auch andere Anwendungen sind jedoch möglich. Der Sensor 10 kann insbesondere ein oder mehrere in den Figuren nicht dargestellte, weitere Funktionselemente umfassen, wie beispielsweise Elektroden, Elektrodenzuleitungen und Kontakte, mehrere Schichten oder andere Elemente. Entsprechend kann der Sensor 10 im Abgasstrang des Wasserstoff-Brennstoffzellenfahrzeugs angebracht werden (exhaust) oder unter atmosphärischen Bedingungen arbeiten (ambient). Folglich kann es sich bei dem Messraum um einen Abgasstrang oder Innenraum des Wasserstoff-Brennstoffzellenfahrzeugs handeln.

Der Sensor 10 weist ein Sensorelement 18 auf. Das Sensorelement 18 weist mindestens eine beheizbare Membran 20 auf. Auf der Membran 20 ist ein erstes Messelement 22 angeordnet. Eine Oberseite 24 der Membran 20 ist einem ersten Referenzraum 26 ausgesetzt, der gegenüber dem Messraum 14 abgeschlossen ist. Der erste Referenzraum 26 ist mit einem fluiden Medium bekannter Zusammensetzung gefüllt. Eine Unterseite 30 der Membran 20 ist dem fluiden Medium 12 aussetzbar.

Das Sensorelement 18 weist weiterhin eine Referenzmembran 32 auf. Auf der Referenzmembran 32 ist ein weiteres Messelement 28 angeordnet. Eine Oberseite 36 der Referenzmembran 32 ist einem zweiten Referenzraum 34 ausgesetzt, der gegenüber dem Messraum 14 abgeschlossen ist. Der zweite Referenzraum 34 ist mit einem fluiden Medium bekannter Zusammensetzung gefüllt. Eine Unterseite 42 der Referenzmembran 32 ist einem dritten Referenzraum 38 ausgesetzt - dies kann dasselbe Medium wie im Referenzraum 34 sein - und von dem fluiden Medium 12 abgetrennt, beispielsweise mittels einer Medientrennung 44. Es wird explizit betont, dass das erste Messelement 22 und das zweite Messelement 28 einstückig ausgebildet sein können. Es wird explizit betont, dass die Referenzmembran 32 optional ist. Der gesamte Aufbau kann beispielsweise in Form eines Siliziumchipwafers ausgeführt werden.

Figur 2 zeigt eine schematische Darstellung eines elektrischen Schaltbilds des Sensorelements 18 des Sensors 10. Dargestellt ist das Sensorelement 18 mit der Membran 20 und der Referenzmembran 32. An das erste Messelement 22 ist eine elektrische Heizspannung U_{H} zum Beheizen der Membran 20 anlegbar (ebenso an das Messelement 28 zum Beheizen der Referenzmembran 32). Die Heizspannung U_{H} wird von einer Spannungsquelle 46 geliefert. Die Membran 20 und die Referenzmembran 32 sind mit einer elektrischen Messbrücke 48 verbunden. Die Messbrücke 48 ist beispielsweise eine Wheatstone'sche Brücke. Die Messbrücke 48 wird dazu beispielsweise von vier Widerständen 50 gebildet, von denen zwei Widerstände 50 auf der Membran 20 und zwei Widerstände 50 auf der Referenzmembran 32 angeordnet sind. An zwei Punkten zwischen den Widerständen 50 der Membran 20 und der Referenzmembran 32 eine elektrische Brückenspannung U_{B} der Messbrücke 48 abgreifbar.

Nachstehend wird ein erfindungsgemäße Verfahren zum Kalibrieren des Sensors 10 beschrieben. An das erste Messelement 22 wird eine elektrische Heizspannung U_{H} zum Beheizen der Membran 20 angelegt. Zusätzlich kann dadurch auch die Referenzmembran 32 durch das zweite Messelement 28 beheizt werden. Dabei stellen sich infolge der unterschiedlichen Wärmeleitfähigkeit der Gase, die die Membran 20 und die Referenzmembran 32 kontaktieren, unterschiedliche Temperaturen im Bereich der Membran 20 und der Referenzmembran 32 ein.

Die Heizspannung U_{H} wird über einen vorbestimmten Zeitraum variiert. Weiterhin wird die elektrische Brückenspannung U_{B} an der Messbrücke 48 über den vorbestimmten Zeitraum gemessen. Bevorzugt wird die Heizspannung U_{H} stetig und periodisch variiert.

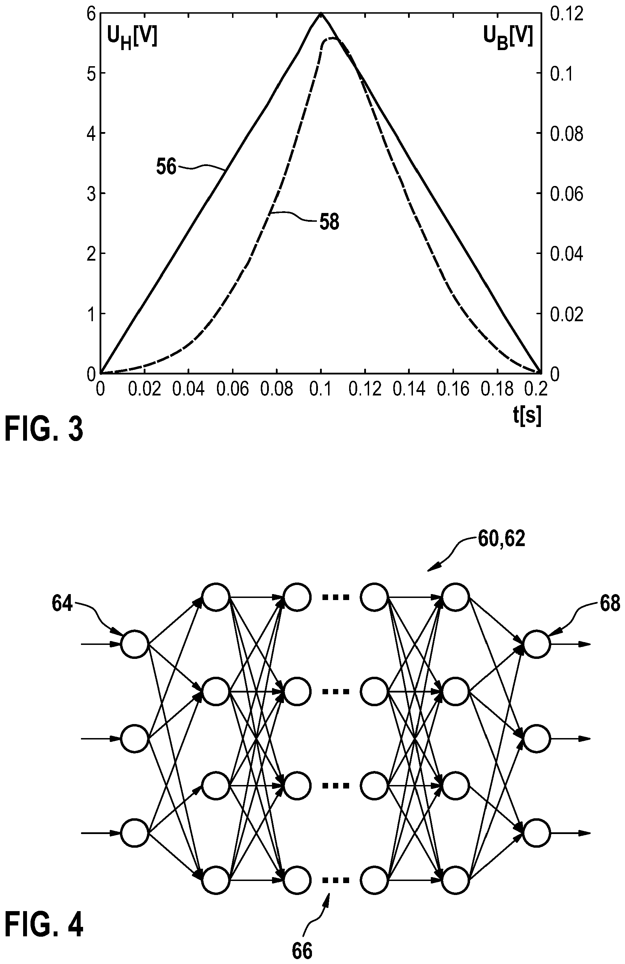

Figur 3 zeigt einen beispielhaften zeitlichen Verlauf von Heizspannung U_{H} und Brückenspannung U_{B}. Auf der X-Achse ist die Zeit t in s aufgetragen. Auf der linken Y-Achse ist die angelegte Heizspannung U_{H} in V aufgetragen. Auf der rechten Y-Achse ist die Brückenspannung U_{B} in V aufgetragen. Die Kurve 56 stellt den zeitlichen Verlauf der Heizspannung U_{H} dar. Die Kurve 58 stellt den zeitlichen Verlauf der Brückenspannung U_{B} dar. Lediglich beispielhaft ist in Figur 3 ein Dreieckssignal für die Heizspannung U_{H} und das dabei resultierende Signal der Brückenspannung U_{B} bei einer bestimmten Gaszusammensetzung dargestellt. Der zeitliche Verlauf des Messsignals enthält viel mehr Informationen als Messungen bei einigen wenigen diskreten Heizspannungen wie beispielsweise über die Gaszusammensetzung und geometrische und elektrische Toleranzen.

Die elektrische Brückenspannung U_{B} wird nach einem Einschwingen eines die Brückenspannung U_{B} anzeigenden Messsignal gemessen. Eine Länge des vorbestimmten Zeitraums wird basierend auf einer physikalischen Zeitskala des Messraums 14 bestimmt. Die physikalische Zeitskala wird basierend auf einer geometrischen Längenskala, Dichte, spezifische Wärmekapazität und Wärmeleitfähigkeit des Messraums 14 bestimmt. Die Länge des vorbestimmten Zeitraums ist 2 bis 20 mal und bevorzugt 5 bis 15 mal größer als die physikalische Zeitskala, beispielsweise 10 mal größer. Durch geeignete Wahl der Periodendauer der Messung abgeleitet von der physikalischen Zeitskala erhält man rasch, z.B. nach einer Periode, einen eingeschwungenen Zustand und damit einen quasi stationären Verlauf der Brückenspannung U_{B}. Die physikalische Zeitskala kann man für das thermische System aus geometrischer Längenskala, Dichte, spezifischer Wärmekapazität und Wärmeleitfähigkeit des Messraums 14 abschätzen: τ = l² ρ cₚ/λ Die Periodendauer für die Messung beträgt dann sinnvollerweise das ca. 10-fache. Genaueres kann man aus Simulationen oder Messungen ableiten. Eine zu kurze Signalperiode ergibt einen geringen Signalhub und eine zu große maximale Membrantemperatur. Eine zu lange Periode erhöht dagegen unnötig die gesamte erforderliche Messzeit.

Weiterhin wird der zeitliche Verlauf der gemessenen Brückenspannung U_{B} mittels eines Algorithmus 60 ausgewertet. Figur 4 zeigt eine schematische Darstellung eines beispielhaften Algorithmus 60 zur Verwendung bei dem erfindungsgemäßen Verfahren. Der Algorithmus 60 umfasst ein künstlich neuronales Netz 62. In Figur 4 ist eine Eingangsschicht 64 des künstlich neuronalen Netzes 62 dargestellt, in der der zeitliche Verlauf der gemessenen Brückenspannung U_{B} als Eingangsgröße eingegeben wird. Weiterhin umfasst das künstlich neuronale Netz 62 mehrere mit der Eingangsschicht 64 verbundene, versteckte Schichten 66. Weiterhin umfasst das künstlich neuronale Netz 62 eine Ausgangsschicht 68, die mit den verdeckten Schichten 66 verbunden ist und die Ausgabegröße liefert. Die Ausgangsgröße ist beispielsweise eine H₂-Konzentration. Somit kann ein funktionaler Zusammenhang zwischen der Eigenschaft des fluiden Mediums 12 und des ausgewerteten zeitlichen Verlaufs der gemessenen Brückenspannung U_{B} ermittelt werden.

Mit anderen Worten wird aus den gesamten Messdaten, d.h. Spannungsverlauf U_{B,ik}(t) für das Teil i und den Versuch k und die sich daraus ergebende Wasserstoff-Konzentration y_{H2,k}, das neuronale Netz 62 trainiert. Dieses bildet somit einen gemessenen Spannungsverlauf auf eine Wasserstoff-Konzentration ab, d.h. U_{B}(t) und die sich daraus ergebende Wasserstoffkonzentration y_{H2}. Da hierbei über den Zeitverlauf sehr viele Stützstellen vorliegen, umfasst das Modell nicht nur die Separation der Gas-Komponenten, sondern auch die Teile-Teile-Streuung -ja sogar die Temperatur des Systems. Und die erforderliche Spannungsgenauigkeit ist ebenfalls geringer als bei der diskreten Messung. In der Fertigung erübrigt sich dadurch eine weitere Kalibrierung, bzw. es genügen einige wenige Validierungsmessungen. Diese wiederum können dazu genutzt werden, das neuronale Netz 62 weiter zu trainieren.

Der Trainingserfolg des künstlich neuronalen Netzes 62 kann weiterhin durch Messungen der Brückenspannung U_{B} bei unterschiedlichen Gaszusammensetzungen des fluiden Mediums 12, wie beispielsweise H₂, O₂, H₂O usw., und/oder unterschiedlichen Temperaturen gesteigert werden.

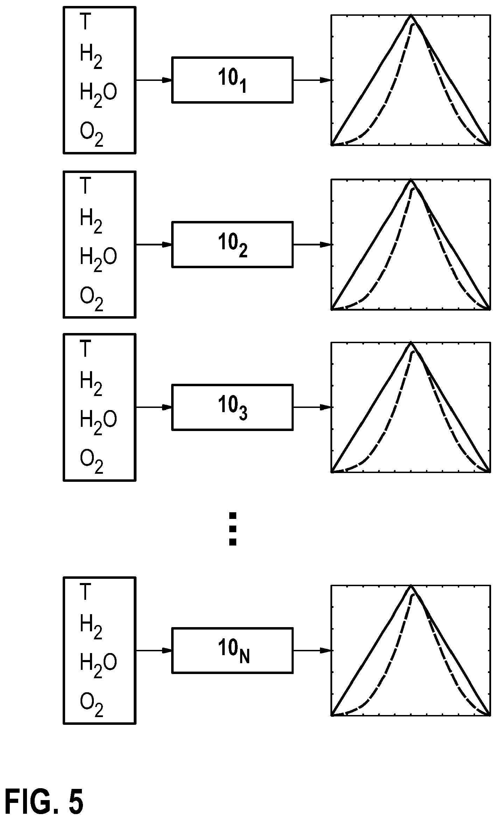

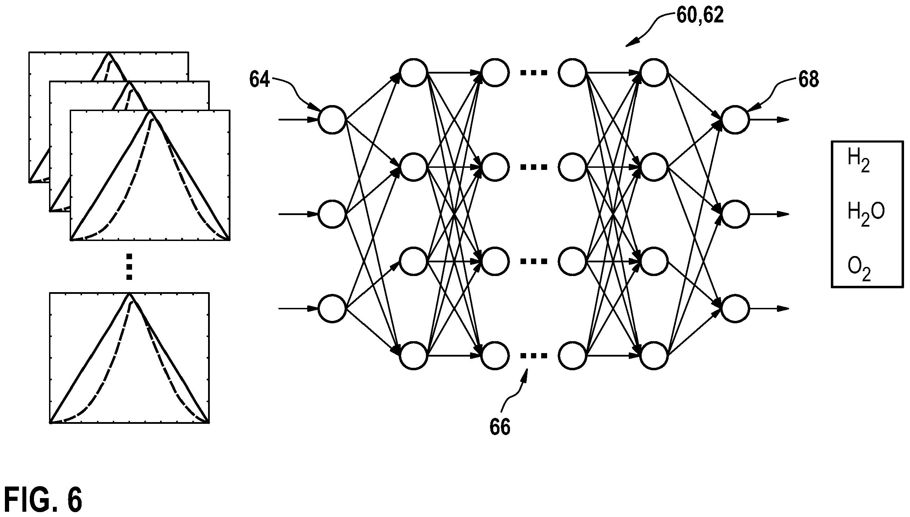

Figur 5 zeigt einen Teil einer Modifikation des erfindungsgemäßen Verfahrens. Figur 6 zeigt einen weiteren Teil der Modifikation des erfindungsgemäßen Verfahrens. Nachstehend werden lediglich die Unterschiede zu dem zuvor beschriebenen Verfahren der Figuren 3 und 4 beschrieben und gleiche oder vergleichbare Bauteile und Merkmale sind mit gleichen Bezugszeichen versehen.

Wie in Figur 5 dargestellt, können bei mehreren verschiedene Sensoren 10 der jeweils die Brückenspannung U_{B} bei variierender Heizspannung U_{H} über den vorbestimmten Zeitraum gemessen werden. Die verschiedenen Sensoren sind der Einfachheit halber in Figur 10 mit den Indices 1, 2, 3 und N dargestellt. Wie weiter dargestellt, können die Messungen der Brückenspannung U_{B} bei unterschiedlichen Gaszusammensetzungen des fluiden Mediums 12, wie beispielsweise H₂, O₂, H₂O usw., und/oder unterschiedlichen Temperaturen durchgeführt werden.

Wie in Figur 6 dargestellt, kann der zeitliche Verlauf der Brückenspannung U_{B} der jeweiligen Sensoren 10₁, 10₂, 10₃, 10_{N} dem Algorithmus 60 bzw. künstlich neuronalen Netz 62 als Eingangsgrößen zugeführt. Als Ausgangsgröße wird dann entsprechend ein genauer Wert für die Konzentration an H₂, O₂, H₂O erhalten. Somit kann der funktionale Zusammenhang zwischen der Eigenschaft eines fluiden Mediums 12 und des ausgewerteten zeitlichen Verlaufs der gemessenen Brückenspannung U_{B} für jeden Sensor 10₁, 10₂, 10₃, 10_{N} ermittelt werden. Dieser funktionale Zusammenhang kann dann als Kennfeld oder dergleichen jedem Sensor 10₁, 10₂, 10₃, 10_{N} hinzugefügt werden. Diese Modifikation stellt eine Untersuchung einer größeren Anzahl von Teilen mit guter Abdeckung der Toleranzen dar, wobei die Werte der toleranzbehafteten Größen, wie beispielsweise Membrandicke, für das Modell des neuronalen Netzes 62 nicht bestimmt werden müssen.

Durch die höhere Informationsdichte beim Training des neuronalen Netzes 62 mittels der Eingangsgröße in Form des zeitlichen Verlaufs der Brückenspannung U_{B} und der zugehörigen Konzentration an beispielsweise Wasserstoff berücksichtigt das Modell des neuronalen Netzes 62 auch den Einfluss der Toleranzen, wie beispielsweise Teile-Teile-Streuung, d.h. der trainierte Zusammenhang liefert für unterschiedliche Teile trotzdem eine gute Genauigkeit für H₂. Daher ist eine aufwendige weitere Kalibrierung während der Serienfertigung nicht mehr notwendig. Lediglich stichprobenartige Kontrollmessungen empfehlen sich. Diese könnten sogar zur Verbesserung des Trainings des neuronalen Netzes 62 (Nachtrainieren) genutzt werden.

Das erfindungsgemäße Verfahren ist durch Einblick in die Steuergeräte-Programmierung und/oder den Kalibrier-Vorgang nachweisbar.

## Patentansprüche

1. Verfahren zum Kalibrieren eines Sensors (10) zur Erfassung mindestens einer Eigenschaft eines fluiden Mediums (12) in mindestens einem Messraum (14), insbesondere zur Erfassung eines H₂-Anteils in einem Messgas (16), wobei der Sensor (10) ein Sensorelement (18) mit mindestens einer beheizbaren Membran (20) aufweist, wobei die Membran (20) mit einer elektrischen Messbrücke (48) verbunden ist, wobei das Verfahren die folgenden Schritte umfasst:
- Anlegen einer elektrischen Heizspannung (U_{H}) an die Membran (20) zum Beheizen der Membran (20),
- Variieren der Heizspannung (U_{H}) über einen vorbestimmten Zeitraum,
- Messen einer elektrischen Brückenspannung (U_{B}) an der Messbrücke (48) über den vorbestimmten Zeitraum,
- Auswerten des zeitlichen Verlaufs der gemessenen Brückenspannung (U_{B}) mittels eines Algorithmus (60), und
- Ermitteln eines funktionalen Zusammenhangs zwischen der Eigenschaft eines fluiden Mediums (12) und des ausgewerteten zeitlichen Verlaufs der gemessenen Brückenspannung (U_{B}).

2. Verfahren nach dem vorhergehenden Anspruch, wobei die Heizspannung (U_{H}) stetig variiert wird.

3. Verfahren nach einem der vorhergehenden Ansprüche, wobei die Heizspannung (U_{H}) periodisch variiert wird.

4. Verfahren nach einem der vorhergehenden Ansprüche, wobei der Algorithmus (60) ein künstlich neuronales Netz (62) umfasst.

5. Verfahren nach einem der vorhergehenden Ansprüche, wobei die elektrische Brückenspannung (U_{B}) nach einem Einschwingen gemessen wird.

6. Verfahren nach einem der vorhergehenden Ansprüche, wobei eine Länge des vorbestimmten Zeitraums basierend auf einer physikalischen Zeitskala des Messraums (14) bestimmt wird.

7. Verfahren nach dem vorhergehenden Anspruch, wobei die physikalische Zeitskala basierend auf einer geometrischen Längenskala, Dichte, spezifische Wärmekapazität und Wärmeleitfähigkeit des Messraums (14) bestimmt wird.

8. Verfahren nach dem vorhergehenden Anspruch, wobei die Länge des vorbestimmten Zeitraums 2 bis 20 mal und bevorzugt 5 bis 15 mal größer als die physikalische Zeitskala ist.

9. Verfahren nach einem der vorhergehenden Ansprüche, wobei die Schritte bei unterschiedlichen Temperaturen in dem Messraum (14) wiederholt werden.

10. Verfahren nach einem der vorhergehenden Ansprüche, wobei das Verfahren bei mehreren verschiedenen Sensoren (10₁, 10₂, 10₃, 10_{N}) durchgeführt wird, wobei der zeitliche Verlauf der gemessenen Brückenspannung (U_{B}) der verschiedenen Sensoren (10₁, 10₂, 10₃, 10_{N}) mittels des Algorithmus (60) ausgewertet werden, wobei der funktionale Zusammenhang zwischen der Eigenschaft eines fluiden Mediums und des ausgewerteten zeitlichen Verlaufs der gemessenen Brückenspannung (U_{B}) für jeden Sensor (10₁, 10₂, 10₃, 10_{N}) ermittelt wird.
